Europäisches Patentamt

European Patent Office

Office européen des brevets

(11) Publication number: **0 667 149 A1**

# EUROPEAN PATENT APPLICATION

(21) Application number: **94830055.3**

(22) Date of filing: **14.02.94**

(51) Int. Cl.6: **A61K 31/00**, A61K 31/19, A61K 9/00

(43) Date of publication of application:
**16.08.95 Bulletin 95/33**

(84) Designated Contracting States:
**AT BE CH DE DK ES FR GB GR IE IT LI LU MC NL PT SE**

(71) Applicant: **E-PHARMA S.p.A**
**Via Provina, 2**
**I-38040 Trento Ravina (TN) (IT)**

(72) Inventor: **Visentin, Fernanda**
**Via G. Marconi, 85**
**I-38010 Grumo S. Michele All'Adige (TN) (IT)**

(74) Representative: **Marietti, Giuseppe et al**
**MARIETTI e GISLON S.r.l.**
**Via Larga, 16**
**I-20122 Milano (IT)**

(54) **Ibuprofen based effervescent composition.**

(57) A pharmaceutical composition based on ibuprofen substantially free of additives, comprising a plurality of basic compounds and one or more acid compounds which form an effervescent couple to solubilize the ibuprofen, in the following proportion:
2 parts by weight of ibuprofen,
3.5 to 12 parts by weight of $NaHCO_3$,
3 to 9 parts by weight of $KHCO_3$,
1.7 to 3.0 parts by weight of $Na_2CO_3$ and
from 2.8 to 6.5 parts by weight of one or more acidic compounds forming the said effervescent couple.

EP 0 667 149 A1

Rank Xerox (UK) Business Services
(3.10/3.09/3.3.4)

The present invention relates to an ibuprofen-based effervescent pharmaceutical composition for oral administration.

More particularly, the invention relates to an effervescent pharmaceutical composition based on ibuprofen, or a pharmaceutically acceptable salt thereof, in granular form, or in tablets obtained from such granules.

Ibuprofen, or 2-(4-isobutylphenyl)propionic acid, has been known for some time to have anti-inflammatory, anti-pyretic and analgesic activities. The molecule has been described e.g. in U.K. Patent No. 971700.

The present state of the art includes numerous applications for ibuprofen-based effervescent pharmaceutical compositions since this form is known to have many advantages in terms of the uptake and bio-availability of the product. This is particularly so in the case of ibuprofen, which presents some problems in oral administration in non-effervescent form, due to the bitter taste of the active principle and the phenomena of intestinal wall attack.

The European Patent Application No. EP-A-0228164 describes an effervescent composition which in addition to ibuprofen and the acid and base components of the effervescent couple also contains water-insoluble hydrophilic polymers and surfactant substances. The drawback of such compositions is essentially that the presence of additives decreases the solubility and bio-availability of the active principle, without obtaining complete solubilization.

The British application GB-A-2189994 relates to a composition in which from 17 to 33% arginine was used to solubilize the active principle. The disadvantage of this is that arginine is an expensive product, and is not involved in the effervescent couple. Furthermore, the tablets produced are relatively large.

European Patent Application EP-A-0369228 describes an effervescent composition where a soluble salt of ibuprofen is used, and that also uses excipients and stabilizers, in particular polyvinylpyrrolidone and similar compounds. The tablets obtained according to this patent weigh about 2.5 g. The main drawback of this composition is the large quantity of expensive stabilizer - 80% of the ibuprofen salt by weight - which interferes with the bio-availability of the active principle.

European Patent Application EP-A-0352353 discloses a composition which is substantially free of stabilizers or surfactants. However, this composition does not give rapid solubilization of ibuprofen since it takes five minutes to solubilize 92% of the active principle. Furthermore, it requires 4.3 g of excipients, flavour and the acid-base couple of the effervescent system to solubilize 200 mg of active principle, thus bringing the weight of the tablet up to 4.5 g.

It is objective of the present invention to produce an effervescent ibuprofen-based composition which allows the size and weight of the tablet to be reduced, gives short solubilization times (circa 2 minutes), is stable in time and substantially free of excipients and dissolving agents.

This objective is achieved by the present invention which relates to an ibuprofen-based effervescent composition characterized according to in Claim 1.

According to a preferred aspect of the invention, the acid used in the effervescent couple is citric or tartaric acid.

According to another preferred aspect of the invention, the composition also includes ascorbic acid (Vitamin C).

According to a further preferred aspect of the invention, the composition consists of up to 3% by weight, and generally between 1.5‰ and 3‰, of monopalmitate sucrose. Preferred composition ranges of the composition according to the invention are as follows : 2 parts by weight of ibuprofen; 3.9 to 10.2 parts by weight of $NAHCO_3$; 3.9 to 8.1 parts by weight of $KHCO_3$; 1.9 to 2.3 parts by weight of $NA_2CO_3$ and 3.4 to 5.6 parts by weight of acid compounds. In place of ibuprofen, the composition may contain the equivalent amount of a pharmaceutically acceptable ibuprofen salt; e.g. 2 parts by weight of ibuprofen can be substituted by 2.213 parts by weight of ibuprofen sodium salt.

The preferred form of the composition according to the invention is granules or tablets obtained from such granules.

The granules are obtained by a process already known in the art, e.g. by granulating the active principle together with the basic compounds of the effervescent couple and granulating the acid component separately and later mixing all together with the flavour. Alternatively, all the components except the flavour are granulated together and afterwards mixed with the flavour.

This technique is known, e.g., from Journal of Pharmaceutical Sciences, 53, (1964) 1524-1525.

Preferably, granulation is effected on ibuprofen particles of average size between 20 and 100 micron and especially 30 to 50 micron.

The invention will now be disclosed with reference to the following compositions according to the invention.

**Example 1**.

Production of 18 mm diameter tablets weighing 2.5 g

Composition of tablet (in mg) :

| Ibuprofen | 200 mg |
|---|---|
| NaHCO$_3$ | 500 mg |
| KHCO$_3$ | 700 mg |
| Na$_2$CO$_3$ | 220 mg |
| Citric Acid | 540 mg |
| Aspartame | 70 mg |
| Sorbitol | 200 mg |
| Flavour | 67 mg |
| Monopalmitate Sucrose | 3 mg |

To produce 1000 tablets, mix 200 g of ibuprofen with 220 g of Na$_2$CO$_3$, 500 g of sodium bicarbonate and 700 g of potassium bicarbonate and suspend over a fluid bed. This mixture is granulated with an aqueous solution of 3 g of Monopalmitate sucrose. The citric acid is granulated separately with an aqueous solution of 270 g of sweeteners.

The two granules are then mixed in a dry mixer together with 67 g of pharmaceutically acceptable flavour, such as raspberry, orange, cherry or mint. The mixture obtained is compressed into 2.5 g tablets of diameter 18 mm (200 mg ibuprofen).

The tablets thus obtained dissolve within 2 minutes in 150 ml of water at 16-18°C, giving a solution of ibuprofen with pH of circa 7.3 with optimum palatability.

**Example 2**.

The process of Example 1 is followed in this example but the quantities of the mixture are modified as in Claim 11 and this gives a composition per tablet of:

| Ibuprofen | 200 mg |
|---|---|
| NaHCO$_3$ | 1000 mg |
| KHCO$_3$ | 400 mg |
| Na$_2$CO$_3$ | 200 mg |
| Citric Acid | 520 mg |
| Aspartame | 50 mg |
| Sorbitol | 5 mg |
| Flavour | 75 mg |
| Saccharine | 30 mg |

The tablets thus obtained dissolve within 2 minutes in 150 ml of water at 16-18°C without stirring, giving a solution of ibuprofen with pH of circa 7.4 with optimum palatability.

**Example 3.**

To obtain 1000 sachets with the following composition:

| Ibuprofen | 200 mg |
|---|---|
| NaHCO$_3$ | 500 mg |
| KHCO$_3$ | 700 mg |
| Na$_2$CO$_3$ | 220 mg |
| Citric Acid | 375 mg |
| Aspartame | 52 mg |
| Saccharine | 30 mg |
| Flavour | 80 mg |
| Monopalmitate sucrose | 3 mg |

200 g of ibuprofen are mixed with the remainder of the compounds, except the flavour, in the ratios listed in Claim 12, and suspended over a fluid bed where the mixture is granulated by spraying with water. The granules are then mixed with the flavour (80 g) and packed into sachets containing 2.16 g, whose contents dissolve within 1 minute in 100 ml of water at 16-18°C without stirring, giving a solution of ibuprofen with pH of circa 7.4 and optimum palatability.

**Example 4**.

The process of Example 3 is repeated with the compounds and quantities as listed in Claim 8 except ascorbic acid, which is mixed with the granules at the same time as the flavour. The granules obtained are compressed into 2.5 g tablets of diameter 18 mm with the following composition:

| Ibuprofen | 200 mg |
|---|---|
| Ascorbic Acid | 225 mg |
| NaHCO$_3$ | 500 mg |
| KHCO$_3$ | 700 mg |
| Na$_2$CO$_3$ | 220 mg |
| Citric Acid | 375 mg |
| Aspartame | 80 mg |
| Sorbitol | 117 mg |
| Flavour | 83 mg |

The tablets thus obtained dissolve within 2 minutes in 150 ml of water at 16-18°C without stirring, giving a solution of ibuprofen and ascorbic acid with pH of circa 7.6 and optimum palatability.

**Example 5**.

The process of Example 4 is followed with the compounds and quantities as listed in Claim 9. The granules obtained are compressed into 2.5 g tablets of diameter 18 mm with the following composition:

| Ibuprofen | 200 mg |
|---|---|
| Ascorbic Acid | 225 mg |
| NaHCO$_3$ | 567 mg |
| KHCO$_3$ | 700 mg |
| Na$_2$CO$_3$ | 220 mg |
| Citric Acid | 375 mg |
| Aspartame | 80 mg |
| Sorbitol | 55 mg |
| Saccharine | 3 mg |
| Flavour | 75 mg |

which dissolve in 150 ml of water at 16-18°C without stirring, within 2 minutes, giving a clear solution of ibuprofen and ascorbic acid with pH of circa 7.5 andoptimum palatability.

**Example 6.**

The process of Example 1 is followed, using the quantities as listed in Claim 13, to obtain tablets with the following composition:

| Ibuprofen | 200 mg |
|---|---|
| NaHCO$_3$ | 800 mg |
| KHCO$_3$ | 600 mg |
| Na$_2$CO$_3$ | 200 mg |
| Citric Acid | 540 mg |
| Aspartame | 50 mg |
| Sorbitol | --- |
| Saccharine | 35 mg |
| Flavour | 75 mg |

The tablets thus obtained dissolve within 2 minutes in 150 ml of water at 16-18°C without stirring, giving a solution of ibuprofen with pH of circa 7.4 with optimum palatability.

**Example 7.**

To obtain 1000 sachets with the following composition :

| Ibuprofen | 200 mg |
|---|---|
| NaHCO$_3$ | 400 mg |
| KHCO$_3$ | 800 mg |
| Na$_2$CO$_3$ | 200 mg |
| Citric Acid | 400 mg |
| Aspartame | 50 mg |
| Saccharine | 35 mg |
| Flavour | 75 mg |
| Monopalmitate Saccharide | 3 mg |
| Sorbitol | 37 mg |

200 g of ibuprofen is mixed with the remainder of the compounds according to the ratios listed in Claim 14 and suspended over a fluid bed where the mixture is granulated by spraying with water. The granules are then mixed with the flavour (75 g) and packed into sachets containing 2.2 g, whose contents dissolve within 1 minute in 100 ml of water at 16-18°C without stirring, giving a solution of ibuprofen with pH of circa 7.4 and optimum palatability.

**Example 8.**

The process of Example 7 is followed with the compounds and quantities as listed in Claim 8 except ascorbic acid, which is mixed with the granules together with the flavour. The granules obtained are compressed into 2.5 g tablets of diameter 18 mm with the following composition:

| Ibuprofen | 200 mg |
|---|---|
| Ascorbic Acid | 225 mg |
| NaHCO$_3$ | 600 mg |
| KHCO$_3$ | 750 mg |
| Na$_2$CO$_3$ | 200 mg |
| Citric Acid | 350 mg |
| Aspartame | 80 mg |
| Sorbitol | 92 mg |
| Flavour | 75 mg |
| Monopalmitate Saccharide | 3 mg |

The tablets thus obtained dissolve within 2 minutes in 150 ml of water at 16-18°C without stirring, giving a solution of ibuprofen and ascorbic acid with pH of circa 7.6, with optimum palatability.

All the compositions obtained according to the present invention have shown optimum shelf life, once packed in moisture-impervious containers.

**Claims**

1. Ibuprofen-based composition, of the type comprising an effervescent couple formed by one or more base compounds and one or more acid compounds, characterized in comprising:
   2 parts by weight of ibuprofen,
   3.5 to 12 parts by weight of NaHCO$_3$,
   3 to 9 parts by weight of KHCO$_3$,
   1.7 to 3.0 parts by weight of Na$_2$CO$_3$ and
   2.8 to 6.5 parts by weight of one or more acidic compounds forming the said effervescent couple.

2. Composition according to claim 1, wherein the said acid is citric acid.

3. Composition according to claims 1 or 2, further comprising 1.8 to 3.0 parts by weight of ascorbic acid.

4. Composition according to any claims 1 to 3, including, for every 2 parts by weight of ibuprofen, 3.9 to 10.2 parts by weight of NaHCO$_3$, 3.9 to 8.1 parts by weight of KHCO$_3$, 1.9 to 2.3 parts by weight of Na$_2$CO$_3$ and 3.4 to 5.6 parts by weight of acidic compounds.

5. Composition according to any preceding claim, further comprising up to 0.03 parts by weight of monopalmitate sucrose.

6. Composition according to any preceding claim, 1.5 to 3.6 parts by weight of pharmaceutically acceptable sweeteners and flavour.

7. Composition according to any of the preceding claims, in the form of granules or tablets.

8. Composition according to any preceding claims, comprising 2 parts by weight of ibuprofen, 5 parts by weight of NaHCO$_3$, 7 parts by weight of KHCO$_3$, 2.2 parts by weight of Na$_2$CO$_3$, 3.75 parts by weight of citric acid, 2.25 parts by weight of ascorbic acid and 2.8 parts by weight of sweeteners and flavour.

9. Composition according to any claim 1 to 8, comprising 2 parts by weight of ibuprofen, 5.67 parts by weight of NaHCO$_3$, 7 parts by weight of KHCO$_3$, 2.2 parts by weight of Na$_2$CO$_3$, 3.75 parts by weight of citric acid, 2.25 parts by weight of ascorbic acid and 2.13 parts by weight of sweeteners and flavour.

10. Composition according to any claims 1 to 7, comprising 2 parts by weight of ibuprofen, 5 parts by weight of NaHCO$_3$, 7 parts by weight of KHCO$_3$, 2.2 parts by weight of Na$_2$CO$_3$, 5.4 parts by weight of citric acid, 0.03 parts by weight of monopalmitate sucrose and 3.37 parts by weight of sweeteners and flavour.

11. Composition according to any claim 1 to 7, comprising 2 parts by weight of ibuprofen, 10 parts by weight of NaHCO$_3$, 4 parts by weight of KHCO$_3$, 2.0 parts by weight of Na$_2$CO$_3$, 5.2 parts by weight of citric acid, 1.6 parts by weight of sweeteners and flavour.

12. Composition according to any claim 1 to 7, comprising 2 parts by weight of ibuprofen, 5 parts by weight of $NaHCO_3$, 7 parts by weight of $KHCO_3$, 2.2 parts by weight of $Na_2CO_3$, 3.75 parts by weight of citric acid, 0.03 parts by weight of monopalmitate sucrose and 1.62 parts by weight of sweeteners and flavour.

13. Composition according to any claims 1 to 7, comprising 2 parts by weight of ibuprofen, 8 parts by weight of $NaHCO_3$, 6 parts by weight of $KHCO_3$, 2 parts by weight of $Na_2CO_3$, 5.4 parts by weight of citric acid, and 1.6 parts by weight of sweeteners and flavour.

14. Composition according to any claim 1 to 7, comprising 2 parts by weight of ibuprofen, 4 parts by weight of $NaHCO_3$, 8 parts by weight of $KHCO_3$, 2 parts by weight of $Na_2CO_3$, 4 parts by weight of citric acid, 0.03 parts by weight of monopalmitate sucrose and 1.97 parts by weight of sweeteners and flavour.

15. Composition according to any claim 1 to 7, comprising 2 parts by weight of ibuprofen, 2.25 parts by weight of ascorbic acid, 6 parts by weight of $NaHCO_3$, 7.5 parts by weight of $KHCO_3$, 2 parts by weight of $Na_2CO_3$, 3.5 parts by weight of citric acid, 0.03 parts by weight of monopalmitate saccharide and 2.47 parts by weight of sweeteners and flavour.

16. Composition according to any of the preceding Claims, modified in that ibuprofen is replaced by an equivalent amount of a pharmaceutically acceptable salt of ibuprofen.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | EP-A-0 351 353 (AESCULAPIUS-PHARMA S.A.)<br>* claims 1-4,6,7 *<br>* page 3, line 6 - line 7 *<br>* examples *<br>--- | 1-16 | A61K31/00<br>A61K31/19<br>A61K9/00 |
| Y | EP-A-0 448 895 (SPECTRUM CONSUMER PRODUCTS CO. INC.)<br>* claims 1-3 *<br>* example 6 *<br>--- | 1-16 | |
| Y | WO-A-92 20334 (THE BOOTS COMPANY PLC)<br>* claims 6,7 *<br>* examples 17-18 *<br>----- | 1-16 | |

TECHNICAL FIELDS SEARCHED (Int.Cl.6)

A61K

The present search report has been drawn up for all claims

| Place of search | Date of completion of the search | Examiner |
|---|---|---|
| THE HAGUE | 8 June 1994 | Scarponi, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)